(19) Europäisches Patentamt
European Patent Office
Office européen des brevets

(11) Veröffentlichungsnummer: **0 247 373**
**B1**

(12) **EUROPÄISCHE PATENTSCHRIFT**

(45) Veröffentlichungstag der Patentschrift: **23.01.91**

(51) Int. Cl.⁵: **C 07 D 311/16,** G 01 N 33/52, G 01 N 21/64, C 12 Q 1/34

(21) Anmeldenummer: **87106175.0**

(22) Anmeldetag: **28.04.87**

(54) **7-(Phenylacetamido)-4-alkylcumarine, Verfahren zu ihrer Herstellung sowie ihre Verwendung in Verfahren zur fluorometrischen Bestimmung der Aktivität von Hydrolasen, insbesondere von Penicillin-G-Acylase.**

(30) Priorität: **30.04.86 DE 3614647**

(43) Veröffentlichungstag der Anmeldung: **02.12.87 Patentblatt 87/49**

(45) Bekanntmachung des Hinweises auf die Patenterteilung: **23.01.91 Patentblatt 91/04**

(84) Benannte Vertragsstaaten: **AT BE CH DE ES FR GB GR IT LI LU NL SE**

(56) Entgegenhaltungen:
**EP-A-0 018 112**
**AT-B- 371 460**
**DE-A-2 932 381**

(73) Patentinhaber: **EUROPÄISCHE ATOMGEMEINSCHAFT (EURATOM)**
**Bâtiment Jean Monnet Plateau du Kirchberg**
**L-2920 Luxembourg (LU)**

(72) Erfinder: **Scheper, Thomas, Dr.**
**Roseggerstrasse 13**
**D-3000 Hannover 1 (DE)**
Erfinder: **Weiss, Martina**
**Trakehnerstrasse 19**
**D-3004 Isernhagen 2 (DE)**
Erfinder: **Schügerl, Karl, Prof.Dr.**
**Arnumer Kirchstrasse 31**
**D-3005 Hemmingen 4 (DE)**

(74) Vertreter: **Weinmiller, Jürgen**
**Lennéstrasse 9 Postfach 24**
**D-8133 Feldafing (DE)**

**EP 0 247 373 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue 7-Phenylessigsäure-4-alkyl-coumarinylamide der weiter unten angegebenen allgemeinen Formel (I), insbesondere die neuen Verbindungen 7-Phenylessigsäure-4-methylcoumarinylamid (PMC) und 7-Phenylessigsäure-4-trifluoromethyl-coumarinylamid (PFC), Verfahren zu ihrer Herstellung sowie ihre Verwendung in Verfahren zur fluorometrischen Bestimmung der Aktivität von Hydrolasen, insbesondere Acylasen, speziell von Penicillin-G-Acylase.

Penicillin-G-Acylase wird industriell in großen Mengen benötigt, da es Penicilline unter Bildung der 6-Aminopenicillansäure (6-APA) spaltet. Die 6-Amino-penicillansäure ist ein Ausgangsstoff für eine breite Palette halbsynthetischer Penicilline, die zum Teil mit diesem Enzym synthetisiert werden können. Das Enzym wird durch Mikroorganismen gebildet. Dabei erfolgt die Enzymproduktion beispielsweise in genetisch modifizierten Zellen oder durch Induktion von E. coli-Stämmen mit Phenylessigsäure.

Die bei der enzymatischen Spaltung von Penicillin-G frei werdenden Protonen lassen sich titrimetrisch für die Aktivitätsbestimmung nutzen. Es ist auch möglich, die gebildete 6-Amino-penicillansäure mit p-Dimethylaminobenzaldehyd (p-DMABA) reagieren zu lassen unter Bildung einer gelb gefärbten Schiffschen Base, die sich photometrisch nachweisen bzw. bestimmen läßt.

Ein anderer photometrischer Test beruht auf der enzymatischen Spaltung der 6-Nitro-3-phenylacetamido-benzoesäure (NIPAB). Die bei der Spaltung entstehende 3-Amino-6-nitrobenzoesäure läßt sich photometrisch bestimmen.

Diese bekannten Verfahren sind jedoch wenig geeignet für die Bestimmung geringer Aktivitäten von Penicillin-G-Acylase.

Man war daher bestrebt, ein Fluoreszenzsubstrat für die Penicillin-G-Acylase zu finden, das folgenden Bedingungen genügt:

a) Das Spaltprodukt der enzymatischen Reaktion sollte sich in seinen Fluoreszenzeigenschaften von denen des Substrats möglichst stark unterscheiden,

b) da Penicillin-G-Acylase ein breites Wirkungsspektrum für Phenylessigsäureamide hat, sollte das Substrat einen Phenylessigsäurerest aufweisen, da dieser für die Entwicklung einer Aktivität gegenüber den Substraten wichtig zu sein scheint; und

c) die Fluoreszenzanregung sollte im UV-Bereich (Quecksilberlampe) oder im Bereich eines Argon-Ionenlasers (488 nm) liegen.

Gesucht wurde deshalb nach einem fluoreszierenden Amin, das sich leicht mit Phenylessigsäure umsetzen läßt und zu einem Produkt führt, für das das Enzym eine ausreichende Aktivität aufweist.

Aus der US—PS—3 741 876 ist ein fluorometrisches Verfahren zur Bestimmung der Aktivität eines Enzyms bekannt, bei dem ein Enzym und ein festes Substrat, das auf eine inerte Siliconmatrix aufgebracht ist, miteinander umgesetzt werden unter Bildung eines fluoreszierenden Materials, wobei die Änderung der Fluoreszenzx mit dem Ablauf der Zeit gemessen wird zur Bestimmung der Konzentration eines der beiden Reaktanten. Als Enzyme können in diesem Verfahren eingesetzt werden Dehydrogenasen, Oxidasen, Phosphatasen, Uricase, Cholinesterase und Lipase.

Aus der EP 0037 583 ist ein Verfahren zur fluorometrischen Bestimmung der Aktivität von Fett abbauenden Enzymen in diese Enzyme enthaltenden Proben bekannt, bei dem die Probe mit einem Substrat kombiniert wird, das eine Verbindung enthält, die mit dem zu testenden Enzym reagiert, das Substrat bei einer spezifischen Anregungswellenlänge der betreffenden fluoreszierenden Gruppen angeregt wird und die Änderung der Fluoreszenzintensität des Substrats unter der Einwirkung des Enzyms pro Zeiteinheit gemessen wird bei einer spezifischen Emissionswellenlänge der fluoreszierenden Gruppe, wobei das Ausmaß der Änderung direkt proportional zur Enzymaktivität der Probe ist. Als Substrate werden in diesem Falle Triacylglycerine verwendet, die durch Lipase in freie Fettsäuren, Glyceride und Glycerin aufgespalten werden.

Aus der EP 0018 112 ist ein Verfahren zur Bestimmung der Anwesenheit eines Enzyms in einer biologischen Flüssigkeit bekannt, bei dem die Flüssigkeit mit einer synthetischen chromogenen Substanz in Kontakt gebracht wird, die das Substrat enthaltende Flüssigkeit inkubiert wird, um die enzymatische Hydrolyse zu bewirken, und der Chromophor in dem dabei erhaltenen Hydrolysat fluorometrisch bestimmt wird. Als chromogenes Substrat wird bei diesem bekannten Verfahren ein Aminosäurederivat von 7-Amino-4-trifluoromethylcoumarin verwendet. Dieses Verfahren eignet sich in erster Linie zur Bestimmung von Proteinase-Enzymen.

Auch diese bekannten Verfahren sind nicht geeignet für den qualitativen und quantitativen Nachweis von Penicillin-G-Acylase auf fluorometrischem Wege, insbesondere bei geringer Aktivität der Penicillin-G-Acylase.

Aufgabe der Erfindung war es daher, chromophore Substrate zu finden, welche die obengenannten Voraussetzungen (a) bis (c) erfüllen und somit geeignet sind für den qualitativen und quantitativen Nachweis der Penicillin-G-Acylase, insbesondere bei geringer Aktivität derselben.

Es wurde nun gefunden, daß diese Aufgabe erfindungsgemäß gelöst werden kann mit neuen 7-Phenylessigsäure-4-alkylcoumarinylamiden der nachstehend angegebenen Formel (I), die hervorragend geeignet sind für den qualitativen und quantitativen fluorometrischen Nachweis von Penicillin-G-Acylase und anderen Hydrolasen, insbesondere anderen Acylasen.

Gegenstand der Erfindung sind neue 7-Phenylessigsäure-4-alkyl-coumarinylamide der allgemeinen Formel

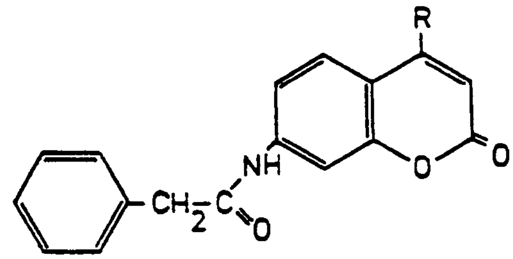

(I)

worin R Alkyl mit 1 bis 4, insbesondere 1 bis 2 Kohlenstoffatomen bedeutet, das gegebenenfalls durch ein oder mehr Halogenatome, insbesondere Fluoratome, substituiert ist.

Besonders vorteilhafte Verbindungen sind solche der vorstehend angegebenen allgemeinen Formel (I), worin R Methyl oder Trifluoromethyl bedeutet.

Die erfindungsgemäßen 7-Phenylessigsäure-4-alkyl-coumarinylamide ergeben bei der enzymatischen Reaktion mit Hydrolasen, insbesondere Acylasen, speziell mit Penicillin-G-Acylase, Spaltprodukte, die sich in ihren Fluoreszenzeigenschaften von denen des Substrats stark unterscheiden und deren Fluoreszenzanregung im UV-Bereich (Quecksilberlampe) oder im Bereich eines Argon-Lasers (488 nm) liegt.

Besonders geeignet sind in diesem Zusammenhang 7-Phenylessigsäure-4-methyl-coumarinylamid (PMC) und 7-Phenylessigsäure-4-trifluoromethyl-coumarinylamid (PFC), die von Penicillin-A-Acylase nach dem folgenden Reaktionsschema gespalten werden:

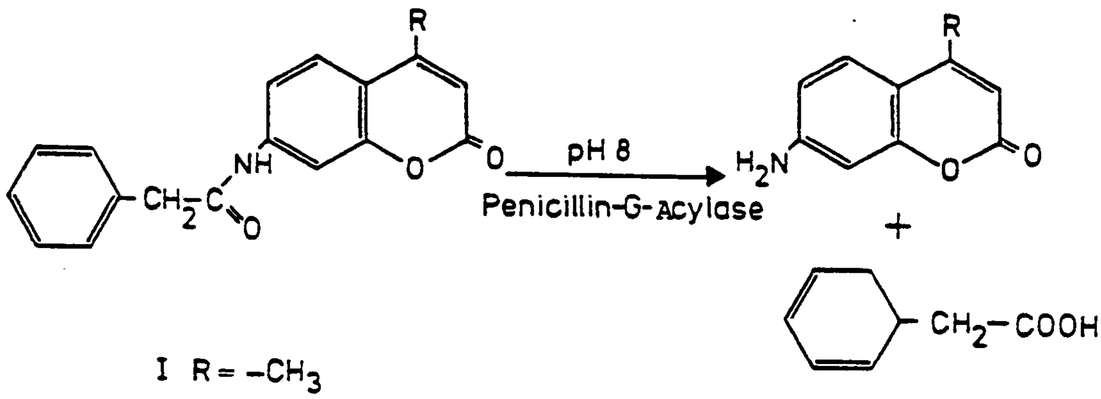


I $R = -CH_3$

II $R = -CF_3$

wobei die freien Aminocoumarine entstehen. Diese Reaktion läßt sich bei pH 8 in 50 mM Kaliumphosphatpuffer durchführen und in einem Spektralfluorometer (Schoeffel) verfolgen. Als Anregungswellenlänge kann für beide Substrate 365 nm genutzt werden. Die Emissionswellenlänge für den enzymatischen Test mit PMC beträgt 440 nm, für den Test mit PFC beträgt sie 495 nm.

Wie aus der Abbildung 1 der beiliegenden Zeichnungen hervorgeht, unterscheiden sich die Substrate und die Reaktionsprodukte sehr stark voneinander. Die Diagramme zeigen die Emissionsspektren des Testgemisches (3 µg/ml Substrat, pH = 8, 50 mM K-P-Puffer (Anregung 365 nm)).

Beide Substrate können in einem Fluoroeszenz-Enzym-Test eingesetzt werden, in dem die zeitliche Zunahme der Fluoreszenzintensität bei verschiedenen Penicillin-G-Acylase-Konzentrationen ermittelt wird. Die dabei erzielten Ergebnisse sind in den Abbildungen 2 und 3 der beiliegenden Zeichnungen dargestellt.

Gegenstand der Erfindung ist ferner ein Verfahren zur Herstellung der 7-Phenylessigsäure-4-alkyl-coumarinylamide der vorstehend angegebenen allgemeinen Formel (I), das dadurch gekennzeichnet ist, daß ein 7-Amino-4-alkylcoumarin der allgemeinen Formel

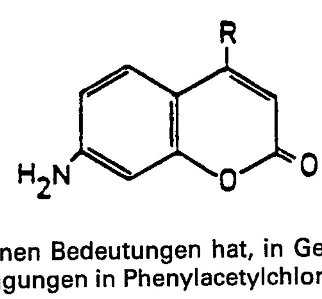

(II)

worin R die weiter oben angegebenen Bedeutungen hat, in Gegenwart einer Base, d.h. eines Protonenakzeptors, unter wasserfreien Bedingungen in Phenylacetylchlorid in Lösung umgesetzt wird und aus dem

dabei erhaltenen Reaktionsgemisch das 7-Phenylessigsäure-4-alkyl-coumarinylamid ausgefällt und gereinigt wird.

Gemäß einer bevorzugten Ausgestaltung der Erfindung wird ein 7-Amino-4-alkyl-coumarin der oben angegebenen Formel (II), worin R Methyl oder Trifluoromethyl bedeutet, in Dioxan gelöst und unter Rühren mit einem geringen Überschuß Pyridin versetzt, wonach Phenylacetylchlorid in geringem Überschuß, ebenfalls gelöst in Dioxan, zugetropft wird. Das erhaltene Reaktionsgemisch wird 3 h gerührt und anschließend auf Eis gegossen, danach mit HCl leicht angesäuert, filtriert und so lange mit destilliertem Wasser gewaschen, bis das Filtrat neutral ist, und das als Produkt erhaltene Amid wird anschließend aus Ethanol umkristallisiert und getrocknet.

Zur Durchführung des erfindungsgemäßen Verfahrens können beliebige Lösungsmittel verwendet werden, wie sie beispielsweise im "Organikum"* unter dem Abschnitt "Säureamidherstellung" beschrieben sind. Die Lösungsmittel müssen für die Herstellung absolut wasserfrei sein, zur Ausfällung des Produkts aber mit Wasser mischbar sein.

Bei der Durchführung des erfindungsgemäßen Verfahrens können beliebige Basen als Protonenempfänger neben Pyridin verwendet werden (vgl. "Organikum", Abschnitt Säureamide).

Die Konzentration, in der die Ausgangsmaterialien in dem Lösungsmittel jeweils gelöst werden, hängt von der Löslichkeit des jeweiligen Fluorophors ab, wobei eine 10%ige Lösung bevorzugt ist. Eine optimale Konzentration bei PMC und PFC beträgt 1 mMol in 60 ml Dioxan.

Die Konzentration an Protonenampfänger (Pyridin) und Phenylacetylchlorid ist mindestens äquimolar, vorzugsweise etwas größer, optimal wird ein 1,1-facher stöchiometrischer Überschuß verwendet.

Der Temperaturbereich zur Durchführung des erfindungsgemäßen Verfahrens ist nicht besonders kritisch. Im allgemeinen liegt er zwischen dem Festpunkt und dem Siedepunkt des Lösungsmittels, wobei jedoch die Zersetzungstemperatur der Reaktionspartner zu beachten ist. Vorzugsweise wird eine Temperatur von 15 bis 35°C, insbesondere von 20 bis 25°C, angewendet.

Die Reaktionszeit beträgt im allgemeinen 10 min bis 24 h, vorzugsweise 30 min bis 6 h, insbesondere 3 h.

Bei der Durchführung des erfindungsgemäßen Verfahrens wird in einem Zwei-Hals-Kolben mit Trockenrohr und Tropfvorrichtung in einem Wasserbad bei 20 bis 25°C das Alkylcoumarin vorgelegt, unter Rühren mit einem Magnetrührer wird Dioxan dazugegeben, dann wird Pyridin zugegeben, wobei sich als Niederschlag ein Pyridin-Aminocoumarin-Komplex bildet. Anschließend wird dann das Acetylchlorid, gelöst in Dioxan, zugetropft.

Die Synthese muß unter absolut wasserfreien Bedingungen durchgeführt werden, da sonst das eingesetzte Acetylchlorid hydrolysiert. Das erhaltene Reaktionsgemisch wird auf die gleiche Menge Eis gegeben, es wird 30%ige HCl zugetropft, bis kein Niederschlag mehr gebildet wird. Nach dem Auflösen des Eises wird erneut HCl zugegeben, bis kein Niederschlag mehr gebildet wird, dann wird, zweckmäßig mittels einer Nutsche, abfiltriert, mit eiskaltem Wasser gespült und aus Ethanol zweimal umkristallisiert und anschließend scharf getrocknet.

Die bei der Durchführung dieses Verfahrens erhaltenen Verbindungen sind licht- und hydrolyseempfindlich und können durch ihre IR- und NMR-Spektren identifiziert werden.

Gegenstand der Erfindung ist ferner die Verwendung der 7-Phenylessigsäure-4-alkyl-coumarinylamide der vorstehend angegebenen Formel (I), insbesondere die Verwendung von 7-Phenylessigsäure-4-methyl-coumarinylamid (PMC) und von 7-Phenylessigsäure-4-trifluoromethyl-coumarinylamid (PFC), zur fluorometrischen Bestimmung der Aktivität von Hydrolasen, insbesondere Acylasen, speziell von Pencillin-G-Acylase.

Ein einen weiteren Gegenstand der Erfindung bildendes fluorometrisches Verfahren zur Bestimmung der Aktivität von Hydrolasen, insbesondere von Penicillin-G-Acylase, ist dadurch gekennzeichnet, daß ein 7-Phenylessigsäure-4-alkyl-coumarinylamid der Formel (I) in einem geeigneten Lösungsmittel gelöst und mit einem Puffer bis auf eine Konzentration von 1 bis 10 µg/ml, vorzugsweise von 2 bis 6 µg/ml, insbesondere von 3 µg/ml, verdünnt wird, die dabei erhaltene Mischung in einem Spektralfluorometer (λ 440 bis 495 nm) auf das Grundsignal vermessen wird, daß dann durch Zugabe von 10 bis 100 µml der zu untersuchenden Testmischung die Reaktion gestartet und im Spektralfluorometer verfolgt wird, wobei aus der linearen Anfangssteigung des Fluoreszenz/Zeit-Diagramms die Enzymaktivität bestimmt wird. Der bei der Durchführung des Tests eingehaltene pH-Wert beträgt im allgemeinen 7,0 bis 9,5, vorzugsweise 7,5 bis 9,0, optimal 8,0 bis 8,5. Als Puffer kann jeder beliebige Puffer verwendet werden, wobei ein K-P-Puffer oder ein Trispuffer besonders bevorzugt ist. Die Konzentration des erfindungsgemäß verwendeten 7-Phenylessigsäure-4-alkyl-coumarinylamids, insbesondere der Verbindungen PFC und PMC, bei Durchführung des fluorometrischen Tests beträgt im allgemeinen 10 bis 250 mMol, vorzugsweise 30 bis 100 mMol, speziell 50 mMol, wobei die Puferkapazität zu beachten ist. Im übrigen ist die Substratkonzentration von der Löslichkeit und den kinetischen Daten abhängig, für PMC und PFC liegt sie zweckmäßig zwischen 1 und 10 µg/ml, vorzugsweise zwischen 2 und 6 µg/ml, ganz besonders bevorzugt bei 3 µg/ml.

Gegenstand der Erfindung ist ferner ein Verfahren zum Nachweis der Aktivität von intracellulärer Hydrolase, insbesondere Penicillin-G-Acylase, das dadurch gekennzeichnet ist, daß die Hydrolase-haltigen Mikroorganismenzellen aus ihrem Kulturmedium abgetrennt und einer Lösung eines 7-Phenylessigsäure-

*"Organikum", VEB Deutscher Verlag der Wissenschaft, Berlin 1975, 14. Auflage.

4-alkyl-coumarinylamids der Formel (I) zugeführt werden, wonach die durch das in den Zellen angereicherte enzymatische Reaktionsprodukt 7-Amino-4-alkylcoumarin verursachte Fluoreszenzemission in einem Fluoreszenzmikroskop bei 440 bis 495 nm beobachtet wird.

Zur Durchführung dieses Verfahrens können auch sogenannte "Trapping Reagents" verwendet werden, bei denen es sich um Substanzen handelt, welche die Löslichkeit des freigesetzten Fluorophors in den Zellen stark herabsetzen, wie z.B. Salze oder Substanzen, die nach der chemischen Reaktion mit Fluor schwerlösliche Produkte ergeben (vgl. Dolbeare, Smith, "J. Clin. Chem.", 23 (1977), und F. Scrienc, J. Bailey, "3rd Europ. Cong. Biotechn., München, 1984).

Gemäß einem weiteren Gegenstand der Erfindung kann die Aktivität intracellulärer Hydrolasen, insbesondere intracellulärer Penicillin-G-Acylase, dadurch bestimmt werden, daß hydrolasehaltige Mikroorganismenzellen aus dem Kulturmedium abgetrennt werden, in kaltem, 50%igem Ethanol fixiert werden, um die Zellmembran für das Reaksionsprodukt 7-Amino-4-alkyl-coumarin permeabel zu machen, daß 20 µl der Zellsuspension in eine Lösung eines 7-Phenylessigsäure-4-alkyl-coumarinylamids der Formel (I) eingeführt werden und die Intensität der als Folge der Reaktion auftretenden Fluoreszenz, die der Konzentration des in die Lösung permeierten Produkts proportional ist, in einem Spektralfluorometer als Funktion der Zeit gemessen und auf die Enzymaktivität zurückgerechnet wird.

Zur Durchführung dieser erfindungsgemäßen Verfahren können alle Mikroorganismen verwendet werden, in denen Penicillin-G-Acylase nachgewiesen werden kann.

Während bei der Durchführung diese fluorometrischen Verfahren die Anregungswellenlänge bei 380 bis 400 (PMC) bzw. 420 bis 440 (PFC) liegt, liegt die Emissionswellenlänge nach der Enzymzugabe bei PMC bei 400 bis 520 nm, vorzugsweise bei 420 bis 460 nm, speziell bei 440 nm, während sie bei PFC bei 440 bis 550 nm, vorzugsweise bei 480 bis 520 nm, speziell bei 495 nm liegt.

Die Erfindung wird nachstehend unter Bezugnahme auf die beiliegenden Zeichnungen näher erläutert. Es ziegen:

Abbildung 1a und 1b Emissionsspektren der Substrate PMC und PFC in einer Pufferlösung (50 mM Phosphatpuffer, pH 8);

Abbildung 2 und 3 die Eichkurven für die beiden Substrate PMC (PMC: 1 µg/ml, 50 mM Puffer, 0% Ethanol, pH 8) und PFC (PFC: 3 µg/ml, 50 mM Puffer, 5% Ethanol, pH 8) (Anregungswellenlänge 365 nm; Emissionswellenlänge 440 nm für PMC bzw. 492 nm für PFC);

Abbildung 4 das Lineweafer-Burg-plot für den PFC-Test; und

Abbildung 5 den fluorometrischen Test mit Penicillin-G-Acylase-haltigen E. coli-Zellen.

Die Zusammensetzung der Substratlösungen ist in den Diagrammen der beiliegenden Zeichnungen jeweils angegeben. Bei den Versuchen wurden jeweils 3 ml der Substralösung mit 10 µl Enzymlösung versetzt. Die Zunahme der Fluoreszenz (bei den angegebenen Werten) wurde 3 min lang verfolgt. Die zeitliche Zunahme der Fluoreszenz als Funktion der Enzymkonzentration im Testgemisch ist in den Diagrammen aufgetragen.

Für das PMC und das PFC liegen die linearen Bereiche der Eichkurven bei 20 bis zu etwa 3000 µU/ml entsprechend einer Volumenkapazität der injizierten Enzymlösung von 6,0 90 mU/ml.

Die Werte für den PFC-Test sind gut reproduzierbar, da die Substratlösung (5% Ethanol) genauer herzustellen ist. Für Substratlösungen ohne Ethanol benötigt man etwa 24 h, um 1 mg Substrat in 1 l Puffer zu lösen. Der fluorometrische Test eignet sich besonders gut für die Bestimmung geringer Aktivitäten von Penicillin-G-Acylase.

Bei den Versuchen wurde wiederholt festgestellt, daß sich die Umsatzgeschwindigkeit der Reaktion bei höheren Substratkonzentrationen verringert. Es mußten daher Untersuchungen zur Bestimmung der kinetischen Parameter ($V_{max}$ und $K_M$) dieses Reaktionssystems durchgeführt werden.

Die Enzynkonzentration im Test wurde dazu konstant gehalten und die Substratkonzentration (50 mM KP-Puffer, 5% Ethanol für beide Substrate) variiert.

Die Abbildung 4 zeigt den Lineweafer-Burg-Plot für das PFC-Derivat. Der Verlauf der Kurve deutet eindeutig auf eine Substrathemmung hin (vgl. H. U. Bergmeyer, "Grundlagen der enzymatischen Analyse", Verlag Chemie/Weinheim, 1976).

Für einen schnellen und gut meßbaren Umsatz der Phenylessigsäurecoumarinylamide muß daher eine geeignete Substratkonzentration im Testgemisch vorliegen. Für beide Substrate liegt diese Konzentration bei etwa 3 µg/ml (50 mM-KP-Puffer; 5% Ethanol). Die Parameter $V_{max}$ und $K_M$ für die beschriebenen Versuchsbedingungen sind wie folgt:

$$\text{PMC} : V_{max} = 11{,}07 \frac{\mu mol}{1\ min} ; K_M = 83{,}1 \frac{\mu mol}{1}$$

$$\text{PFC} : V_{max} = 3{,}33 \frac{\mu mol}{1\ min} ; K_M = 44{,}7 \frac{\mu mol}{1}$$

Für fluorometrische Tests mit Mikroorganismen ist das PFC-Derivat besonders gut geeignet. Die Fluoreszenzemission liegt nach der enzymatischen Spaltung bei 495 nm, also im sichtbaren (grünen) Bereich. Diese Wellenlänge kann mit einem Fluoreszenzmikroskop (Diawert mit Fluoreszenzzusatz der Firma Leitz) gut beobachtet werden. Mikroorganismen mit intracellulärer Penicillin-G-Acylase-Aktivität

lassen sich sehr einfach und spezifisch mit diesem Substrat anfärben und im Fluoreszenzmikroskop erkenne. Das Reaktionsprodukt 7-Amino-4-(trifluoromethyl)coumarin entsteht intracellulär und wird dort bis zu einem gewissen Grade angereichert.

Um diese Reaktion auch im Spektralfluorometer zu verfolgen, wurden Penicillin-G-Acylase-haltige E. coli-Zellen 10 min lang in kaltem 50%igem Ethanol fixiert. Dabei wurde die Zellwand so stark permeabilisiert, daß sie sowohl für das Substrat als auch für das Produkt der Umsetzung gut durchlässig war. 20 µl der Zellsuspension wurden in eine PFC-Lösung injiziert und fluorometrisch verfolgt (vgl. Abbildung 5).

Die mit der NIPAB-Methode bestimmte Volumenaktivität der Suspension lag erheblich unter 100 mU/ml, die mit der PFC-Methode bestimmte Volumenaktivität lag bei 45 mU/ml. Versuche mit höheren Volumenaktivitäten zeigten, daß beide Methoden gut übereinstimmten.

Die erfindungsgemäßen Coumarinderivate, insbesondere PFC und PMC, sind als Substrate für die fluorometrische Bestimmung von Hydrolasen, insbesondere Acylasen, speziell von Penicillin-G-Acylase, gut geeignet. Die Reaktion, auf der die Bestimmungsmethode beruht, kann durch das folgende Schema dargestellt werden:

nicht-fluoreszierendes Substrat (z.B. PMC, PFC) —Enzym→ fluoreszierendes Produkt + Nebenprodukte

Der optimale Konzentrationsbereich der Substrate (PMC, PFC) im Test liegt bei 3 µg/ml (5% Ethanol). Höhere Konzentrationen sind wegen einer Substrathemmung nicht sinnvoll. Mit dem Test lasen sich auch geringe Konzentrationen des Enzyms — auch intracellulär — gut erfassen. Zur Durchführung dieses Tests können im Prinzip alle Fluorophore mit einer Aminogruppe verwendet werden, wobei jedoch zu beachten ist, daß das Syntheseprodukt ein anderes Fluoreszenzverhalten aufweist als der freie Fluorophor nach der enzymatischen Spaltung.

Die erfindungsgemäße Reaktion ist für Penicillin-G-Acylase spezifisch. Sie kann aber auch auf andere Hydrolasen angewendet werden, wobei dann statt der für Penicillin-G-Acylase spezifische Phenylacetylgruppe die jeweilige spezifische Gruppe dieses Enzyms einzuführen ist.

Die bei der Herstellung der erfindungsgemäßen 7-Phenylessigsäure-4-alkyl-coumarinylamide, insbesondere PFC und PMC, erzielten Ausbeuten lagen bei hoher Produktrienheit (NMR, IR) zwischen 50 und 80%. Die beiden zuletztgenannten Substrate weisen eine geringe Löslichkeit in wäßrigen Lösungen (unter 1 mg/l) auf. Dieses Problem läßt sich jedoch dadurch umgehen, daß die Substrate zuerst in reinem Ethanol gelöst und dann mit dem entsprechenden Puffer auf eine Mischung mit 5% Ethanol verdünnt werden. Auf diese Weise können Konzentrationen von 6 mg/l erzielt werden.

Die Erfindung wird durch die folgenden Beispiele näher erläutert, ohne jedoch darauf beschränkt zu sein.

### Beispiel 1

Herstellung der Coumarinylderivate PMC und PFC

Es wurden die folgenden Chemikalien eingesetzt:

| | |
|---|---|
| 7-Amino-4-methylcoumarin | (der Firma Aldrich Chemical Co.) |
| 7-Amino-4-(trifluoromethyl)-coumarin | (der Firma Aldrich Chemical Co.) |
| Phenylacetylchlorid | (der Firma Fluka) |
| Dioxan, getrocknet | (der Firma Merck) |
| Pyridin, getrocknet | (der Firma Merck) |
| Ethanol | (der Firma Merck) |
| 32%ige Salzsäure | (der Firma Merck) |

In einem Zwei-Hals-Kolben mit Trockenrohr und Tropfvorrichtung, der auf ein Wasserbad von 20 bis 25°C gesetzt wird, wird 1 mMol Coumarin vorgelegt und unter Rühren (mit einem Magnetrührer) werden 60 ml Dioxan zugegeben, um das Coumarin zu lösen. Dann werden unter Rühren 1,1 mMol Pyridin zugegeben, wobei ein Niederschlag des Pyridin-Aminocoumarin-Komplexes entsteht. Anschließend werden 1,1 mMol Phenylacetylchlorid, gelöst in 2 ml Dioxan, innerhalb von 2 min zugetropft. Das Reaktionsgemisch wird 3 h gerührt und anschließend auf die gleiche Menge Eis gegeben, das Gemisch

wird mit 32%iger HCl angesäuert, bis kein Niederschlag mehr entsteht. Nach der Auflösung des Eises wird erneut HCl zugegeben, bis kein Niederschlag mehr entsteht, dann wird mittels einer Nutsche abfiltriert, mit eiskaltem Wasser so lange gewaschen, bis das Filtrat neutral ist. Das dabei erhaltene Amid wird aus Ethanol zweimal umkristallisiert (etwa 150 ml für PMC und etwa 30 ml für PFC) und scharf getrocknet. Die dabei erhaltenen Coumarinylderivate sind wasserfrei und lichtdicht im Gefreierschrank aufzubewahren. Die Ausbeuten betrugen 67 bis 77% für PMC und 52 bis 57% für PFC.

Beispiel 2

Test zur Bestimmung der Aktivität von Penicillin-G-Acylase mit den Coumarinylderivaten PMC und PFC

Die Testmischung enthält 3 µg/ml der Coumarinylderivate, wie sie in Beispiel 1 erhalten werden, in 50 mM Kaliumphosphatpuffer (pH, 8, 5% Ethanol) gelöst.

Zuerst wird eine Lösung der Coumarinylderivate in Ethanol hergestellt und mit dem Phosphatpuffer den Konzentrationangaben entsprechend vedünnt. 3 ml der Mischung werden in einer Fluoreszenz-Küvette in einem Spektralfluorometer (λ = 440 nm für PMC; λ = 495 nm für PFC) auf das Grundsignal vermessen.

Durch Zugabe von 10 bis 100 µl Probe wird die Reaktion gestartet (unter Rühren) und verfolgt. Aus der linearen Anfangssteigung des Fluoreszenz/Zeit-Diagramms kann die Enzymaktivität ermittelt werden, nachdem eine entsprechende Eichung vorgenommen worden ist.

Beispiel 3

Nachweis der Aktivität von intracellulärer Penicillin-G-Acylase

Penicillin-G-Acylase-haltige E. coli-Zellen werden aus dem Nährmedium abgetrennt (beispielsweise durch Zentrifugieren) und in eine PFC-Lösung, wie sie in Beispiel 2 erhalten wurde, eingeführt. Die Fluoreszenzemission, die durch das in den Zellen angereicherte enzymatische Reaktionsprodukt 7-Amino-4-(trifluoromethyl)-coumarin hervorgerufen wird, wird in einem Fluoreszenzmikroskop (der Firma Leitz, Diawert mit Fluoreszenzzusatz) bei 495 nm beobachtet.

Beispiel 4

Bestimmung der Aktivität von intracellulärer Penicillin-G-Acylase

Penicillin-G-Acylase-haltige E. coli-Zellen werden aus dem Nährmedium abgetrennt (beispielsweise durch Zentrifugieren) und 10 min lang in kaltem, 50%igem Ethanol fixiert, um die Zellmembran auch für das Produkt des PFC, nämlich 7-Amino-4-(trifluoromethyl)coumarin, permeabel zu machen.

20 µl der Zellsuspension werden in eine PFC-Lösung, wie sie nach Beispiel 2 erhalten wurde, injiziert und die Fluoreszenzintensität, die der Konzentration des in die Lösung permeierten Produkts proportional ist, wird in einem Spektralfluorometer als Funktion der Zeit gemessen (vgl. Abbildung 5) und auf die Enzymaktivität zurückgerechnet.

Beispiel 5

Bestimmung der Aktivität von intracellulärer Penicillin-G-Acylase

Das in Beispiel 3 beschriebene Verfahren wiord wiederholt unter Verwendung von "Trapping Reagents", d.h. unter Verwendung von Substanzen, welche die Löslichkeit des freigesetzten Fluorophors in den Zellen stark herabsetzen, z.B. Salze (wie NaCl) oder Substanzen, die nach der chemischen Reaktion mit Fluor schwerlösliche Produkte ergeben.

Der nach Durchführung der Reaktion erzielbare hohe Anteil von bis zu 50% Ethanol in der Färbelösung wird nach 30-minütiger Reaktion nach dem Zentrifugieren durch einen 0,9%ige NaCl-Lösung stark verdünnt. Dadurch wird die Löslichkeit des Reaktionsprodukts in den Zellen stark verringert.

**Patentansprüche**

1. 7-Phenylessigsäure-4-alkyl-coumarinylamide, gekennzeichnet durch die allgemeine Formel:

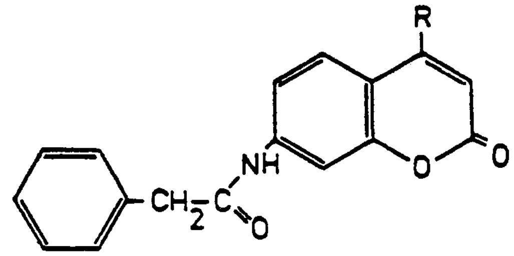

(I)

worin R Alkyl mit 1 bis 4, insbesondere 1 bis 2 Kohlenstoffatomen bedeutet, das gegebenenfalls durch ein oder mehr Halogenatome, insbesondere Fluoratome, substituiert ist.

2. Verbindung nach Anspruch 1, dadurch gekennzeichnet, daß in der allgemeinen Formel (I) R Methyl oder Trifluoromethyl bedeutet.

3. 7-Phenylessigsäure-4-methyl-coumarinylamid (PMC).

4. 7-Phenylessigsäure-4-trifluoromethyl-coumarinylamid (PFC).

5. Verfahren zur Herstellung der 7-Phenylessigsäure-4-alkyl-coumarinylamide nach einem der Ansprüche 1 bis 4, dadurch gekennzeichnet, daß ein 7-Amino-4-alkylcoumarin der allgemeinen Formel

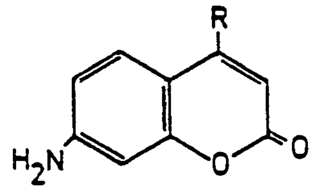

(II)

worin R die in Anspruch 1 angegebene Bedeutung hat, in Gegenwart einer Base (Protonenakzeptor) unter wasserfreien Bedingungen mit Phenylacetylchlorid in Lösung umgesetzt wird und aus dem dabei erhaltenen Reaktionsgemisch das 7-Phenylessigsäure-4-alkyl-coumarinylamid ausgefällt und gereinigt wird.

6. Verfahren nach Anspruch 5, dadurch gekennzeichnet, daß ein 7-Amino-4-methylcoumarin der in Anspruch 5 angegebenen Formel (II), worin R Methyl oder Trifluoromethyl bedeutet, in Dioxan gelöst und unter Rühren mit einem geringen Überschuß Pyridin versetzt wird, wonach Phenylacetylchlorid im Überschuß, gelöst in Dioxan, zugetropft wird, das Reaktionsgemisch 3 h gerührt und anschließend auf Eis gegossen wird, das erhaltene Gemisch mit HCl leicht angesäuert, filtriert und so lange mit destilliertem Wasser gewaschen wird, bis das Filtrat neutral ist, und daß das als Produkt erhaltene Amid anschließend aus Ethanol umkristallisiert und getrocknet wird.

7. Verwendung der 7-Phenylessigsäure-4-alkyl-coumarinylamide nach einem der Ansprüche 1 bis 4 zur fluorometrischen Bestimmung der Aktivität von Hydrolasen, insbesondere von Penicillin-G-Acylase.

8. Fluorometrisches Verfahren zur Bestimmung der Aktivität von Hydrolasen, insbesondere von Penicillin-G-Acylase, dadurch gekennzeichnet, daß ein 7-Phenylessigsäure-4-alkyl-coumarinylamid nach einem der Ansprüche 1 bis 4 in einem geeigneten Lösungsmittel gelöst und mit einem Puffer bis auf eine Konzentration von 1 bis 10 µg/ml verdünnt wird, die dabei erhaltene Mischung in einem Spektralfluorometer (λ 440 bis 495 nm) auf das Grundsignal vermessen wird, daß dann durch Zugabe von 10 bis 100 µl der zu untersuchenden Testmischung die Reaktion gestartet und im Spektralfluorometer verfolgt wird, wobei aus der linearen Anfangssteigung des Fluoreszenz/Zeit-Diagramms die Enzymaktivität bestimmt wird.

9. Verfahren zum Nachweis der Aktivität von intrazellulärer Hydrolase, insbesondere Penicillin-G-Acylase, dadurch gekennzeichnet, daß die Hydrolase-haltigen Mikroorganismenzellen aus ihrem Kulturmedium abgetrennt und einer Lösung eines 7-Phenylessigsäure-4-alkyl-coumarinylamids nach einem der Ansprüche 1 bis 4 zugeführt werden, wonach die durch das in den Zellen angereicherte enzymatische Reaktionsprodukt 7-Amino-4-alkyl-coumarin verursachte Fluoreszenzemission in einem Fluoreszenzmikroskop bei 440 bis 495 nm beobachtet wird.

10. Verfahren zur Bestimmung der Aktivität intrazellulärer Hydrolasen, insbesondere intrazellulärer Penicillin-G-Acylase, dadurch gekennzeichnet, daß Hydrolasehaltige Mikroorganismenzellen aus dem Kulturmedium abgetrennt werden, in kaltem, 50%igem Ethanol fixiert werden, um die Zellmembran für das Reaktionsprodukt 7-Amino-4-alkyl-coumarin permeabel zu machen, daß 20 µl der Zellsuspension in eine Lösung eines 7-Phenylessigsäure-4-alkyl-coumarinylamids nach einem der Ansprüche 1 bis 4 eingeführt werden und die Intensität der als Folge der Reaktion auftretenden Fluoreszenz, die der Konzentration des in die Lösung permeierten Produkts proportional ist, in einem Spektralfluorometer als Funktion der Zeit gemessen und auf die Enzymaktivität zurückgerechnet wird.

**Revendications**

1. 7-(phénylacétamido)-4-alkyl-coumarinylamides, caractérisés par la formule générale

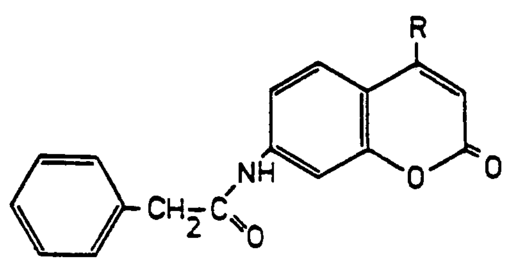

(I)

dans laquelle R indique un alkyl avec 1 à 4, en particulier 1 à 2 atomes de carbone, éventuellement substitués par un ou plusieurs atomes d'halogène, en paritculier des atoems de fluor.

2. Des composés selon la revendication 1, caractérisés par le fait que dans la formule genérale (I) R signifie méthyl ou trifluorométhyl.

3. 7-(phénylacétamido)-4-méthyl-coumarinylamide (PMC).

4. 7-(phénylacétamido)-4-trifluorométhyl-courmarinylamide (PFC).

5. Procédé pour la fabrication des 7-(phénylacétamido)-4-alkyl-coumarinylamides selon une des revendications 1 à 4, caractérisé par le fait qu'un 7-amino-4-alkyl-coumarine de la formule générale

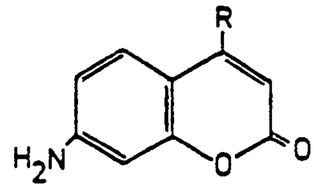

(II)

dans lequel R a les significations indiquées dans la revendication 1, est mis en solution sous des conditions anhydres dans du chlorure de phénylacétyle en présence d'une base, c'est-à-dire d'un accepteur de protons, et que de mélange de réaction en résultant, on laisse précipiter le 7-(phénylacétamido)-4-alkyl-coumarinylamide et on le nettoie.

6. Procédé selon la revendication 5, caractérisé par le fait qu'un 7-amino-4-alkyl-coumarine de la formule (II) citée dans la revendication 5, dans laquelle R désigne méthyl ou trifluorométhyl, est dissout dans du dioxane et un faible surplus de pyridine est ajouté en agitant, après quoi un faible surplus de phénylacétylchloride, de même dissout dans du dioxane, est additionné goutte par goutte, le mélange de réaction ainsi obtenu est agité pendant 3 heures et puis versé sur de la glace, puis légèrement acidifié avec du HCl, filtré et lavé avec de l'eau distillée jusqu'à ce que le filtrat soit neutre, et que l'amide obtenu est alors recristallisé de l'éthanol et séché.

7. Utilisation des 7-phénylacétamido-4-alkyl-coumarinylamide selon l'une des revendications 1 à 4 pour la détermination fluorométrique de l'activité des hydrolases, en particulier des acylases de la pénicilline G.

8. Procédé fluorométrique pour la détermination de l'activité des hydrolases, surtout de l'acylase de la pénicilline G, caractérisé par le fait qu'un 7-(phénylacétamido)-4-alkyl-coumarinylamide selon l'une des revendications 1 à 4 est dissout dans un solvant approprié et dilué à l'aide d'un tampon jusqu'à une concentration de 1 à 10 µg/ml, le mélange obtenu est mesuré sur le signal de base dans un spectrofluorimètre ($\lambda$ = 440 à 495 nm), qu'alors par addition de 10 à 100 µl du mélange de test à analyser la réaction est démarrée et observée dans le spectrofluorimètre, l'activité enzymatique étant déterminée par la pente initiale du diagramme fluorescence/temps.

9. Procédé pour la détection de l'activité d'une hydrolase intracellulaire, en particulier de l'acylase de la pénicilline G, caractérisé par le fait que les cellules des microorganismes contenant de l'hydrolase sont séparées de leur milieu de culture et additionnées à une solution d'un 7-(phénylacétamido)-4-alkyl-coumarinylamide selon l'une des revendications 1 à 4, après quoi l'émission de fluorescence, causée par le produit de réaction 7-amino-4-alkylcoumarine enzymatique enrichi dans les cellules, est observée dans un microscope de fluorescence à 440 à 495 nm.

10. Procédé pour la détection de l'activité des hydrolases intercellulaires, en particulier de l'acylase de la pénicilline G intracellulaire, caractérisé par le fait que des cellules de micro-organismes contenant de l'hydrolase sont séparées du milieu de culture, sont fixées dans de l'éthanol froid à 50%, pour rendre la membrane cellulaire perméable pour le produit de réaction 7-amino-4-alkyl-coumarine, que 20 µl de la suspension cellulaire sont introduits dans une solution d'un 7-(phénylacétamido)-4-alkyl-coumarinylamide selon l'une des revendications 1 à 4 et que l'intensité de la fluorescence qui résulte de la réaction et qui est proportionelle à la concentration du produit ayant pénétré par perméation dans la solution est mesurée en fonction du temps et donne après calcul l'activité enzymatique.

**Claims**

1. 7-(Phenylacetamido)-4-alkyl coumarins, characterized by the general formula:

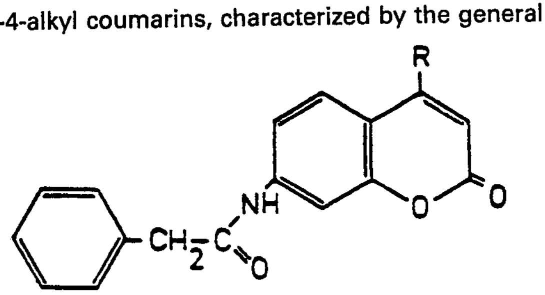

(I)

wherein R is alkyl having 1 to 4, in particular 1 to 2 carbon atoms, optionally substituted by one or more halogen atoms, in particular fluorine atoms.

2. The compounds according to claim 1, characterized in that in general formula (I) R is methyl or trifluoromethyl.

3. 7-(Phenylacetamido)-4-methyl coumarins (PMC).

4. 7-(Phenylacetamido)-4-trifluoromethyl coumarin (PFC).

5. A process for preparing the 7-(Phenylacetamido)-4-alkyl coumarins according to any of claims 1 to 4, characterized in that a 7-amino-4-alkyl coumarin of general formula

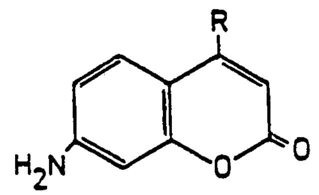

(II)

wherein R has the meaning given in claim 1 is reacted with phenylacetyl chloride in solution in the presence of a base (proton acceptor) under anhydrous conditions and the 7-(Phenylacetamido)-4-alkyl coumarin is precipitated from the obtained reaction mixture and purified.

6. The process according to claim 5, characterized in that a 7-amino-4-methyl coumarin having the formula (II) given in claim 5 wherein R is methyl or trifluoromethyl is dissolved in dioxane and added with a slight excess of pyridine while stirring it, then phenylacetyl chloride, dissolved in dioxane, is added dropwise in excess, the reaction mixture is stirred for 3 hours and afterwards poured onto ice, the obtained mixture is slightly acidified with HCl, filtered and washed in distilled water until the filtrate is neutral, and the obtained amide product finally is recrystallized from ethanol and dried.

7. A use of the 7-(phenylacetamido)-4-alkyl coumarins according to one of claims 1 to 4 for the fluorometric determination of the activity of hydrolases, in particular penicillin-G-acylase.

8. A fluorometric process for the determination of the activity of hydrolases, in particular penicillin-G-acylase, characterized in that a 7-(phenylacetamido)-4-alkyl coumarin according to any of claims 1 to 4 is dissolved in a suitable solvent and diluted with a buffer to a concentration of 1 to 10 µg/ml, the obtained mixture is measured for the basis signal in a spectrofluorometer (λ 440 to 495 nm), then the reaction is started by adding 10 to 100 µl of the test mixture to be measured and followed in the spectrofluorometer whereby the enzyme reactivity is determined from the linear initial gradient of the fluorescence/time diagram.

9. A process for detecting the activity of intracellular hydrolyase, in particular penicillin-G-acylase, characterized in that the hydrolase-containing microorganism cells are separated from their culture medium and are introduced into a solution of a 7-(phenylacetamido)-4-alkyl coumarin according to any of claims 1 to 4 and then the fluorescence emission due to the enzymatic reaction product 7-(phenylacetamido)-4-alkyl coumarin enriched in the cells is observed in a fluorescence microscope at 440 to 495 nm.

10. A process for the determination of the activity of intracellular hydrolases, in particular intracellular penicillin-G-acylase, characterized in that the hydrolase-containing microorganism cells are separated from the culture medium, fixed in cold 50% ethanol in order to make permeable the cell membrane for the reaction product 7-(phenylacetamido)-4-alkyl coumarin, 20 µl of the cell suspension are introduced into a solution of a 7-(phenylacetamido)-4-alkyl coumarin according to any of claims 1 to 4 and the intensity of the fluorescence due to the reaction, the intensity being proportional to the concentration of the product permeated into the solution, is measured in a spectrofluorometer as a function of the time and backcalculated to the enzyme activity.

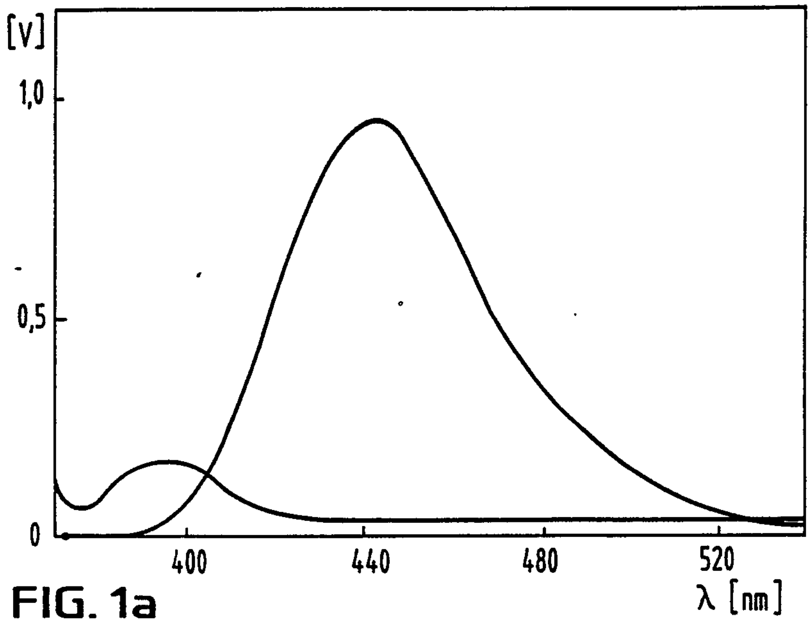


FIG. 1a

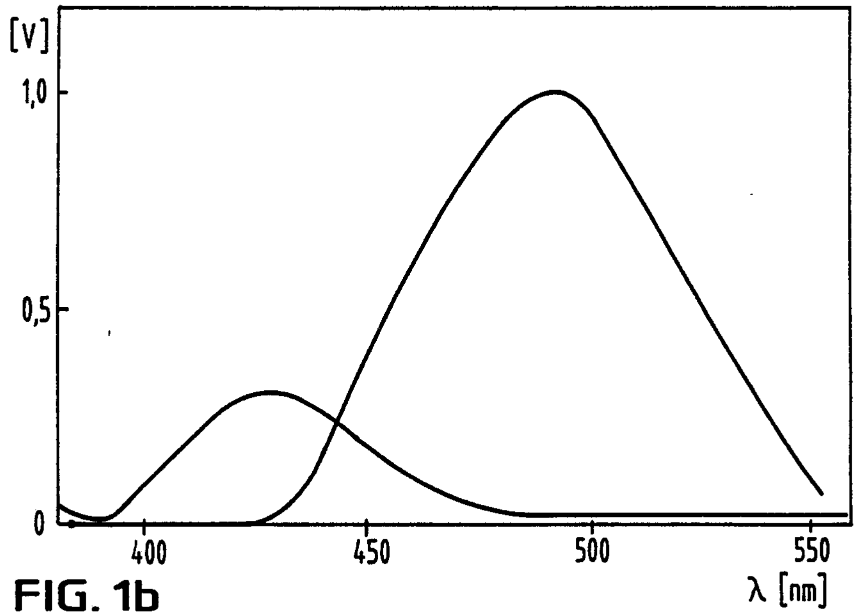


FIG. 1b

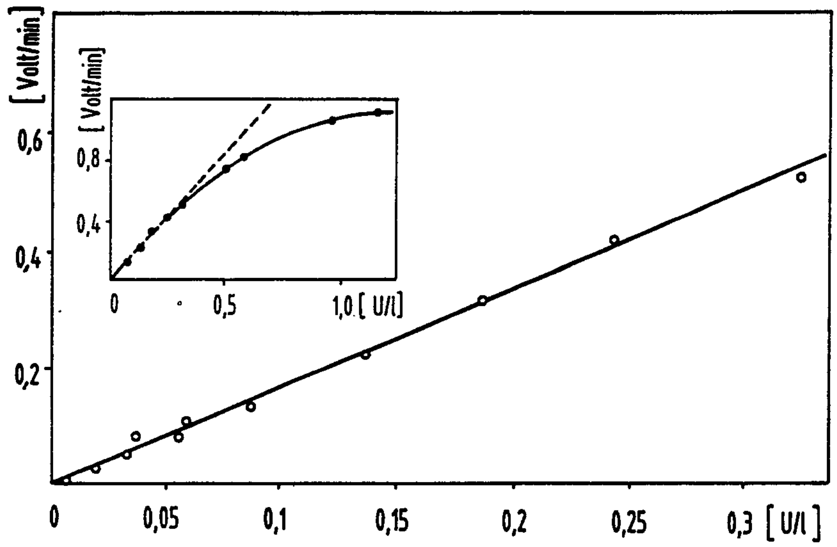
[ Volt/min ]
0,6
0,4
0,2
0
0,05
0,1
0,15
0,2
0,25
0,3 [ U/l ]
[ Volt/min ]
0,8
0,4
0
0,5
1,0 [ U/l ]

FIG. 2

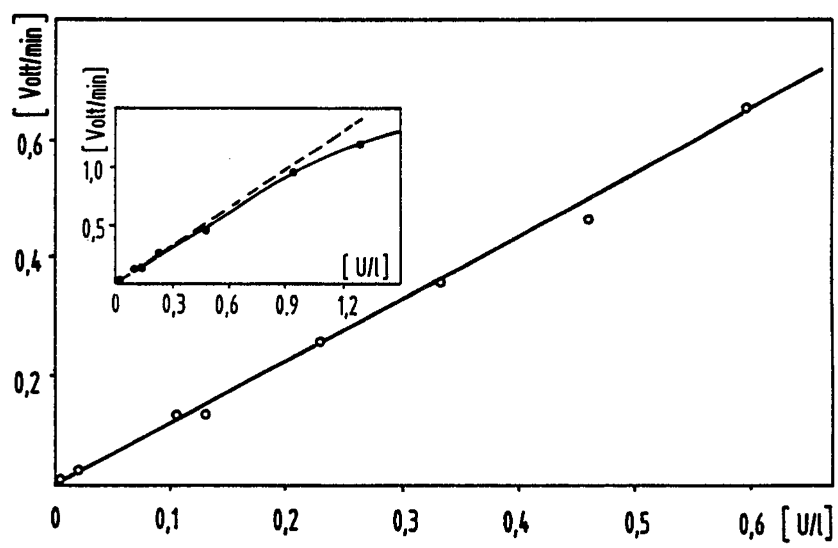
[ Volt/min ]
0,6
0,4
0,2
0
0,1
0,2
0,3
0,4
0,5
0,6 [ U/l ]
[ Volt/min ]
1,0
0,5
[ U/l ]
0
0,3
0,6
0,9
1,2

FIG. 3

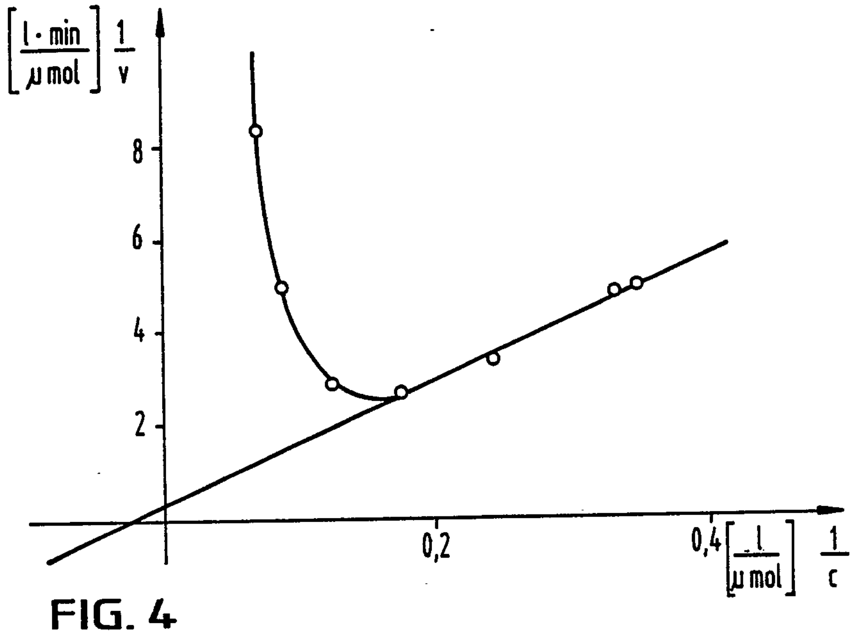


FIG. 4

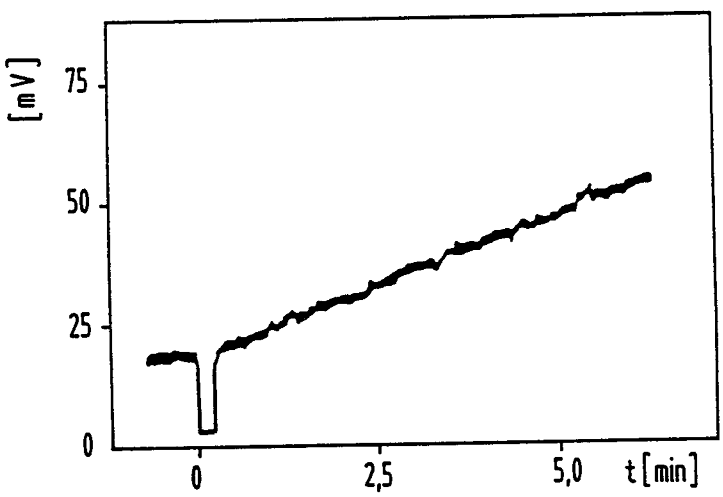


FIG. 5